# EUROPEAN PATENT APPLICATION

(11) **EP 4 215 126 A1**
(43) Date of publication of application: **26.07.2023**
(21) Application number: 21884596.4
(22) Date of filing: 19.08.2021
(51) Int. Cl.: A61B 17/12

(54) **MEDICAL SYSTEM AND MEDICAL DEVICE**

(30) Priority: 28.10.2020 CN 202011172384
(71) Applicant: Shanghai MicroPort CardioAdvent Co., Ltd., Shanghai Pilot Free Trade Zone Shanghai 201203 (CN)
(72) Inventor: REN, Kaibing, Shanghai 201203 (CN); ZHU, Zexun, Shanghai 201203 (CN); LIU, Tianyu, Shanghai 201203 (CN); YAO, Yao, Shanghai 201203 (CN); LI, Junfei, Shanghai 201203 (CN)
(74) Representative: Marks & Clerk LLP
(86) International application number: PCT/CN2021/113546
(87) International publication number: WO 2022/088864

(57) **Abstract**

The present invention relates to a medical system and a medical device. The medical system includes a delivery device (20) and a degradable occluder (10). The occluder (10) comprises a main body (11, 12), a proximal fixing member (14), a distal fixing member (15) and an anchoring portion (16). The main body (11, 12) has a proximal end connected to the proximal fixing member (14), and a distal end connected to the distal fixing member (15). The anchoring portion is defined on the main body. The delivery device (20) comprises an external push tube (21) and an internal push member (22). The external push tube (21) is configured to be detachably connected to the proximal fixing member (14). The internal push member (22) is configured to pass through the external push tube (21), the proximal fixing member (14) and the main body (11, 12) successively to detachably connect to the distal fixing member (15). The delivery device (20) is configured to apply an axial pressure to the occluder (10), to cause the main body (11, 12) to expand from a collapsed configuration to an expanded configuration to drive the anchoring portion (16) to stretch out. The invention solves the problem that the degradable occluder cannot self-expand to a predetermined shape in the body cavity due to the degradable material lacking shape memory ability, and the stabs cannot stretch out autonomously to achieve anchoring at the target position.

## Description

### TECHNICAL FIELD

The present invention relates to the technical field of body cavity occlusion, in particular to a medical system and a medical device.

### BACKGROUND

Atrial fibrillation is the most common sustained cardiac arrhythmia clinically, which has the risk of inducing ischemic stroke. Data show that in patients with nonvalvular atrial fibrillation, more than 90% of cardiogenic thrombus forms in the left atrial appendage. Recent studies have shown that closure of the left atrial appendage can effectively prevent the risk of ischemic stroke caused by atrial fibrillation. Since the first clinical application of left atrial appendage occlusion to prevent thromboembolic events in atrial fibrillation in 2001, its clinical application has developed rapidly at home and abroad, and has become an important method for preventing thromboembolic events in patients with atrial fibrillation. At the same time, different types of left atrial appendage occlusion devices continue to come out, which better meet the clinical needs and improve the efficacy and safety of surgery.

The occluders used for left atrial appendage occlusion in the prior art can basically be divided into two categories, one is the cage-shaped occluder represented by Watchman, which is characterized in that the self-expanding stent is integrally cut and formed, and anchor hooks are arranged circumferentially, and the atrial surface is covered with a porous permeable membrane, which is inserted into the cavity of the left atrial appendage to block it during use. The other is double-disc occluder represented by LAmbre, which is characterized in that it is composed of a positioning disc and an occluding disc which are connected with each other. When in use, the positioning disc is embedded in the left atrial appendage for anchoring, and it may also be used for occlusion to some extent. The occluder mainly relies on the occluding disc configured to be attached to the ostium of the left atrial appendage for occlusion. At present, both the occluders have a common feature, that is, they are mainly made of nickel-titanium alloy, and once implanted in the human body, they will accompany the patient for life. Since this kind of material cannot be degraded, long-term implantation in the human body will cause inflammation, blood coagulation and other reactions with human tissues, and even cause a certain degree of damage.

In addition, there may be the following risks: (1) Nitinol is a non-degradable metal material. Although its biocompatibility has been demonstrated, the long-term risk of permanent implantation cannot be fully predicted and controlled; (2) There is no long-term follow-up data on the safety of the left atrial appendage occluder permanently staying in the heart; (3) There is no clear scientific demonstration of complications such as nickel precipitation and allergies. In addition, when the left atrial appendage ostium is completely endothelialized, the left atrial appendage occluder will lose its function, and there is no need to keep it in the body. Therefore, an ideal LAA (left atrial appendage) occluder should be provided temporarily for the endothelialization of the LAA ostium. After the endothelialization is completed, it will be degraded by the body, so that the LAA occlusion will be completely performed by the tissue of LAA, so as to avoid retention of foreign object, which may cause long-term complications and safety hazards.

Although the existing technology has designed the degradable left atrial appendage occluder, it mainly studies its material and processing technology, and also proposes some certain structural designs. The degradable material has no shape memory ability, and thus the left atrial appendage occluder cannot self-expand to the predetermined shape after being pushed out of the sheath, which affects the penetration of the anchor, resulting in no reliable connection between the occluder and the left atrial appendage, and which makes it easy to fall off, threatening the life of the patient, and cannot effectively occluding the left atrial appendage and thus affects the curative effect of the operation. Not only that, but occluders in A/VSD or other circumstances may have the same problem.

### SUMMARY OF THE INVENTION

In view of the problems existing in the prior art, an object of the present invention is to provide a medical system and a medical device to solve the problem that the existing degradable occluder cannot self-expand to a predetermined shape in the human body due to the degradable material lacking shape memory ability, and thus the anchor cannot autonomously penetrate the target site.

According to a first aspect of the present invention, there is provided a medical system, comprising a delivery device and a degradable occluder;
wherein the occluder comprises a main body, a proximal fixing member, a distal fixing member and an anchoring portion; wherein the main body has a proximal end connected to the proximal fixing member, and a distal end connected to the distal fixing member; the anchoring portion is defined on the main body;
wherein the delivery device comprises an external push tube and an internal push member; wherein the external push tube is configured to be detachably connected to the proximal fixing member; the internal push member is configured to pass through the external push tube, the proximal fixing member and the main body successively to detachably connect to the distal fixing member;
wherein the medical system is configured so that the delivery device applies an axial pressure to the occluder, to cause the main body to expand from a collapsed configuration to an expanded configuration to drive the anchoring portion to stretch out.

Optionally, the main body comprises a degradable metal stent and the anchoring portion integrally formed with the metal stent.

Optionally, the metal stent comprises a plurality of support rods arranged circumferentially; wherein at least one of the support rods are cut at a middle part thereof to form the anchoring portion having a leading end and a tail end which are opposite to each other, wherein the tail end is connected to the metal stent, and the leading end is a free end;
wherein the anchoring portion is formed by cutting from one side of the middle part of the support rod, and/or the middle part of the support rod has a width larger than a width of rest parts of the support rod.

Optionally, the occluder has an initial state and an expanded state;
when the occluder is in the initial state, a negative angle is formed between an extension direction of the anchoring portion and a positive direction of an axis of the main body, and/or the a profile of the anchoring portion along a length direction thereof is arc-shaped;
when the occluder is in the expanded state, a positive angle is formed between the extension direction of the anchoring portion and the positive direction of the axis of the main body, and/or the profile of the anchoring portion along the length direction thereof is arc-shaped.

Optionally, the negative angle ranges from -50° to -30°, and the positive angle ranges from 20° to 40°.

Optionally, the internal push member is a rod or a flexible member.

Optionally, the main body comprises a degradable metal stent and an occluding disc made by braiding degradable polymer material;
wherein the metal stent and the occluding disc are connected through a hollow connection tube; the anchoring portion is arranged on the metal stent; a distal end of the metal stent is connected to the distal fixing member, and a proximal end of the occluding disc is connected to the proximal fixing member.

Optionally, the hollow connection tube is elastic.

Optionally, the hollow connection tube is a spring integrally formed with the metal stent by cutting.

Optionally, a proximal end of the hollow connection tube is defined with a connecting portion, and a hole extending through the connecting portion is defined in the connecting portion; the connecting portion is connected to a distal end of the occluding disc by hot melting.

Optionally, the metal stent and the occluding disc expand simultaneously or sequentially after the occluder receives the axial pressure from the delivery device.

Optionally, the occluder is made of degradable metal material and/or degradable polymer material;
wherein the degradable metal material comprises at least one of magnesium-based metal, zinc-based metal and iron-based metal, and the degradable polymer material comprises polylactic acid, polydioxanone, polycaprolactone, polyglycolide, and polylactide-co-glycolide.

Optionally, the medical system further comprises a limiting device configured to control a size of the expanded occluder.

Optionally, the limiting device comprises a limiting fastener and a limiting slot; when the occluder expands to a predetermined size, the limiting slot engages with the limit fastener.

According to a second aspect of the present invention, there is provided a medical device, made of degradable materials, the medical device comprising a degradable metal stent and an anchoring portion connected with the metal stent;
when the medical device is subjected to axial pressure, the metal stent expands from a collapsed configuration to an expanded configuration to drive the anchoring portion to stretch out.

Optionally, the medical device further comprises an occluding disc made by braiding degradable polymer material; wherein the metal stent and the occluding disc are connected through a hollow connection tube.

Optionally, a ratio of the width of the middle part of the support rod to the width of the rest parts of the support rod is 1.1:1-1.3:1; and/or a ratio of a width of the middle part of the support rod without the anchoring portion to the width of the rest parts of the support rod is 0.85:1-0.95:1.

In the medical system and medical device according to the present invention, the occluder (medical device) can be degraded as a whole, which solves the problems of long-term complications and safety hazards caused by long-term implantation of the occluder. Although the degradable material of the occluder does not have shape memory ability, the delivery device can be used to exert an axial pressure on the occluder to make the occluder expand to a predetermined shape after it moves out of the sheath, so as to achieve an effective occlusion at the target site (where for example a patent foramen ovale, atrial septal defect, ventricular septal defect, patent ductus arteriosus or other congenital heart diseases, etc. may occur), which ensures the effect of occlusion and improves the curative effect of surgery. On another aspect, as the occluder expands, the anchoring portion of the occluder can be driven to stretch out and penetrate into the target site for securement, realizing a stable and reliable connection of the occluder at the target position, improving the reliability and safety of the occluder, and ensuring the safety of the patient's life.

In the medical system and medical device according to the present invention, the occluder preferably includes a degradable metal stent, and the anchoring portion is integrally cut and formed with the metal stent, which not only facilitates forming and anchoring, but also ensures the strength of the occluder. In particular, the anchoring portion can be formed by cutting from one side of the middle part of the support rod of the metal stent, and/or the support rod of the metal stent is designed so that the ratio of the width of the middle part thereof to the width of the rest parts is 1.1:1-1.3:1; and/or the ratio of the width of the middle part of the support rod without the anchoring portion to the width of the rest parts is 0.85:1-0.95:1, thus improving the structural strength of the metal stent, allowing the occluder to be more firmly arranged in the human body.

In the medical system and medical device according to the present invention, before the expansion of the occluder, the anchoring portion is preferably pre-bent to control the shape and angle of the anchoring portion. For example, before the expansion, a negative angle is formed between the extension direction of the anchoring portion and the positive direction of the axis of the occluder, and/or, before the expansion, the profile of the anchoring portion along the length direction is arc-shaped, so that the anchoring portion can be expanded at an appropriate angle after the occluder is expanded, which makes it not easy for the anchoring portion to deviate from the target position, and improves the stability of the connection.

In the medical system and medical device according to the present invention, the occluder preferably includes a degradable metal stent and an occluding disc formed by braiding degradable polymer materials, and the metal stent is connected to the occluding disc by a hollow connection tube, and the anchoring portion is arranged on the metal stent. Such a configuration enhances the fixation performance and occlusion performance of the occluder. Specifically, the occluding disc is formed by braiding degradable polymer materials having the characteristics of softness and good compliance, which helps the occluding disc to better fit the ostium of the body cavity (e.g., the ostium of the left atrial appendage), and thus improves the effectiveness of occlusion. Moreover, the soft occluding disc is beneficial to reduce or even eliminate damage to the ostium of the left atrial appendage and nearby tissues, thereby improving the safety of occlusion. The use of the metal fixing disc with increased strength is beneficial for occlusion and prevents it from falling off.

In the medical system and medical device according to the present invention, the hollow connection tube preferably has an elastic structure, such as a spring or a metal cut tube, so that the occluder can adapt to a body cavity (e.g., the left atrial appendage) whose body and ostium are not coaxial, improving the adaptability of the occluder.

In the medical system and medical device according to the present invention, it is preferable to further include a limiting device configured to control the size of the expanded occluder, so as to precisely control the expansion of the occluder and make the operation simpler and more convenient.

### BRIEF DESCRIPTION OF THE DRAWINGS

Fig. 1a is a schematic structural diagram of an occluder according to a preferred embodiment of the present invention;
Fig. 1b is a front view of the medical system according to a preferred embodiment of the present invention;
Fig. 2a is a schematic diagram showing that the occluder has been pushed out of the sheath, but has not been pushed to expand, and the anchoring portion has not stretched out, according to a preferred embodiment of the present invention;
Fig. 2b is a schematic diagram showing that the occluder is being pushed to expand, and the anchoring portion has been stretched out, according to a preferred embodiment of the present invention;
Fig. 2c is a schematic diagram showing that the occluder has been pushed to expand, the anchoring portion has been stretched out, and the internal push member and the external push tube has not been withdrawn, according to a preferred embodiment of the present invention;
Fig. 3 is a schematic diagram showing the occluder released before expansion according to a preferred embodiment of the present invention;
Fig. 4 is a schematic diagram showing that the occluder is being pushed to expand, and the anchoring portion is being stretched out, according to a preferred embodiment of the present invention;
Fig. 5 is a schematic diagram showing that the occluder has been pushed to expand, the anchoring portion has been stretched out, according to a preferred embodiment of the present invention;
Fig. 6 is a schematic diagram showing that the occluder has been expanded under pressure, the anchoring portion has been stretched out, and the external push tube and the internal push member are removed, according to a preferred embodiment of the present invention;
Fig. 7 is a schematic structural diagram of the anchoring portion of the occluder according to a preferred embodiment of the present invention;
Fig. 8a is a schematic diagram showing a non-prebent anchoring portion of the occluder when the occluder has not been expanded according to a preferred embodiment of the present invention;
Fig. 8b is a schematic diagram showing the non-prebent anchoring portion of the occluder in Fig. 8a after the occluder has been expanded;
Fig. 9a is a schematic diagram showing a pre-bent anchoring portion of the occluder when the occluder has not been expanded according to a preferred embodiment of the present invention;
Fig. 9b is a schematic diagram showing the pre-bent anchoring portion of the occluder in Fig. 9a after the occluder has been expanded;
Fig. 10a is a schematic diagram showing a pre-bent and pre-shaped anchoring portion of the occluder when the occluder has not been expanded according to a preferred embodiment of the present invention;
Fig. 10b is a schematic diagram showing the pre-bent and pre-shaped anchoring portion of the occluder in Fig. 10a after the occluder has been expanded;
Fig. 11 is a schematic structural diagram of a hollow connection tube according to a preferred embodiment of the present invention;
Fig. 12 is a perspective view of the occluder provided with a limiting device according to a preferred embodiment of the present invention.

List of reference numerals:
10, occluder;
11, fixing disc; 111, support rod; 12, occluding disc;
13, hollow connection tube; 131, spring segment; 132, connecting portion; 133, hole;
14, proximal fixing member; 15, distal fixing member; 16, anchoring portion; 161, tail end; 162, leading end;
17, limiting device; 171, limiting slot; 172, limiting fastener;
20, delivery device;
21, external push tube; 22, internal push member;
30, left atrial appendage;
40, liner rod.

### DETAILED DESCRIPTION

To make the objectives, advantages and features of the present invention clearer, the present invention will be further described in detail below with reference to the figures. It is noted that the figures are provided in a very simplified form not necessarily presented to scale, with their only intention to facilitate convenience and clarity in explaining the disclosed embodiments.

It should be noted that the structures, proportions, sizes, etc. shown in the drawings attached to this specification are only used to match the content disclosed in the specification, for those who are familiar with this technology to understand and read, and are not used to limit the implementation of the present invention, so it has no technical substantive meaning, any modification of structure, change of proportional relationship or adjustment of size, without affecting the effect and purpose of the present invention, should still fall within the scope of the present invention. Terms such as "upper", "lower", "left", "right", "middle" and "a" in this specification are only for the convenience of description and are not used to limit the scope of this specification. Any change or adjustment of its relative relationship without causing any substantial change in the technical content shall also be regarded as the practicable scope of the present invention.

Herein, the proximal end refers to the end of the occluder or medical device close to the operator, the distal end refers to the end of the occluder or medical device away from the operator, and the radial direction refers to the direction perpendicular to the axial of the occluder or medical device.

As described in the background, the existing occluders for occluding the left atrial appendage, atrial/ventricular septal defect, etc. are made of biodegradable materials, but the biodegradable materials have no shape memory ability, they cannot self-expand to a predetermined shape after release. This not only adversely affects the reliable connection of the occluder with the target site, and thus makes it easy to cause the occluder to fall off, thereby reducing the safety, but also causes that the target site cannot be effectively occluded, thereby affecting the curative effect of the operation. For example, most of the existing left atrial appendage occluders are formed by cutting (e.g., Watchman Occluder) or braiding (e.g., Lambre Occluder) nickel-titanium alloy materials. Once implanted, they will permanently remain in the human heart, which will bring unknown long-term risks and security risks. Although the degradable occluder can avoid these problems, the currently proposed degradable occluder does not solve the problem in the connection between the occluder and the left atrial appendage, and the problem of inability to restore the predetermined shape after being pushed out, resulting in ineffective occlusion.

In order to solve the above technical problems, the present invention proposes a novel medical system including an occluder and a delivery device. The occluder can be applied to the left atrial appendage, and can also be applied to circumstances involving for example patent foramen ovale, atrial septal defect, ventricular septal defect, patent ductus arteriosus, or other congenital heart diseases. The present invention also proposes a medical device including a degradable metal stent and an anchoring portion connected to the metal stent, the anchoring portion is also degradable and preferably integrally cut and formed with the metal stent.

In order to illustrate the technical solution provided by the present invention, the left atrial appendage occluder is taken as an applicable example below. The occluder provided by the present invention can not only be degraded to avoid long-term complications and potential safety hazards caused by long-term implantation of the occluder, but also be expandable to a predetermined shape to ensure the performance of occluding the left atrial appendage, and also realizes a stable and reliable connection between the occluder and the left atrial appendage, which improves the reliability and safety of the occluder and ensures the safety of the patient's life. More specifically, the occluder of the present invention is configured to be pushed out of the sheath after being delivered to a desired position in the human body by the delivery device, and thereafter, the occluder is controlled to be pressurized and expanded by means of the delivery device, so as to expand to a predetermined shape, and when the occluder expands, it can also drive the anchoring portion thereof to strentch out and penetrate into the inner wall of the left atrial appendage. It should be known that the occluder according to the present invention may be a cage-shaped insertable occluder, or a double-disc occluder, and the double-disc occluder may be used for external or internal occlusion.

The occluder is made of a biodegradable material, such as a degradable polymer material or a degradable metal material. If the occluder is implemented as a cage-shaped insertable occluder, the occluder only includes a cage-shaped stent, which is integrally formed by cutting a degradable metal material or a degradable polymer material, preferably a degradable metal tube. If the occluder is implemented as a double-disc occluder, the occluder includes a fixing disc and an occluding disc, which can be processed in a same way or in different ways. In addition, the fixing disc is generally formed as a cage-shaped stent by cutting a degradable metal tube, and the occluding disc can be formed by integrally cutting or braiding degradable metal materials or degradable polymer materials. Preferably, the occluding disc is formed by braiding degradable polymer materials. Degradable metal materials include, but are not limited to, magnesium-based metals (such as magnesium alloys), zinc-based metals (zinc alloys), or iron-based metals (iron alloys). Degradable polymer materials include, but are not limited to, polylactic acid, polydioxanone, polycaprolactone, polyglycolide, and polylactide-co-glycolide. It should be known that the occluder can be made of one or a combination of degradable metal materials, and/or one or a combination of degradable polymer materials. In other words, the whole occluder can be made merely of degradable metal material(s) or degradable polymer material(s), or can be made of degradable metal material(s) and degradable polymer material(s). It should also be known that, in practical use of the double-disc occluder, the fixing disc and the occluding disc can be inserted as a whole into the inner cavity of the left atrial appendage to achieve occlusion, or only the fixing disc can be inserted into the inner cavity of the left atrial appendage while the occluding disc is positioned at the outer ostium for occlusion, or in an atrial septal defect, the fixing disc and the occluding disc can be positioned at both sides of the atrial septum for occlusion in treating the atrial septal defect.

Whether the occluder is implemented as a double-disc occluder or a cage-shaped insertable occluder, the occluder according to the present invention should include a main body made of degradable material, a proximal fixing member, a distal fixing member, and an anchoring portion. The main body has a proximal end connected with the proximal fixing member, a distal end connected with the distal fixing member, and an anchoring portion is defined on the main body. If the occluder is implemented as a cage-shaped insertable occluder, the main body preferably includes a cage-shaped stent formed by cutting a metal tube, and the proximal and distal ends of the cage-shaped stent are respectively provided with a proximal fixing member and a distal fixing member. If the occluder is implemented as a double-disc occluder, the main body includes an occluding disc and a fixing disc, the distal end of the occluding disc and the proximal end of the fixing disc are connected by a hollow connection tube, and a distal fixing member is arranged at the distal end of the fixing disc, and a proximal fixing member is arranged at the proximal end of the occluding disc.

In addition, the delivery device includes an external push tube and an internal push member. The external push tube is configured to detachably connect with the proximal fixing member. The internal push member is configured to pass through the external push tube, the proximal fixing member and the main body to detachably connect with the distal fixing member. In practical use, when the occluder is delivered to the desired position by the delivery device and pushed out from the sheath, as long as one of the external push tube and the internal push member remains still, the other moves in a predetermined direction (i.e., proximally or distally), the occluder expands from the collapsed configuration to the expanded configuration after being subjected to axial pressure. During the expansion process of the occluder, the anchoring portion that is initially retracted is also gradually stretched out and finally penetrates the inner wall of the left atrial appendage. After it is ensured that a firm connection is established between the occluder and the left atrial appendage, the internal push member and the external push tube can be withdrawn successively to complete the occlusion of the left atrial appendage.

In order to make the above objects, features and advantages of the present invention more comprehensible, preferred embodiments of the present invention will be described in detail below in conjunction with the accompanying drawings. For the sake of brevity, in the following description, it is assumed that the occluder is implemented as a double-disc occluder, and those skilled in the art should be able to modify the following description, and apply the description to a cage-shaped insertable occluder after making appropriate modifications on the details. For the sake of explanation, in the following description, it is assumed that the occluder is implemented as a left atrial appendage occluder, and those skilled in the art should be able to modify the following description, and use the description for a device other than a left atrial appendage occluder after making appropriate modifications in details.

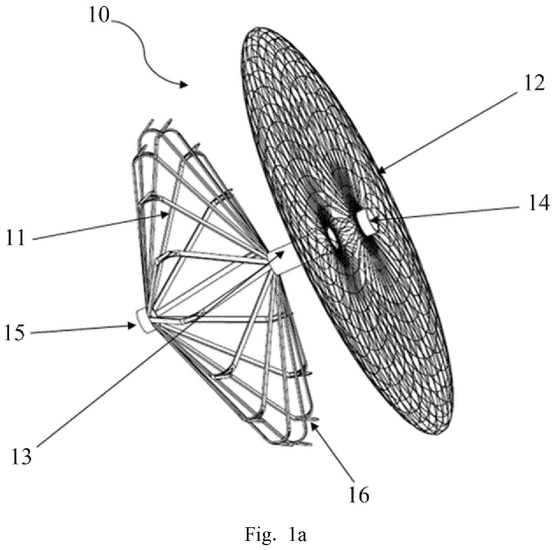

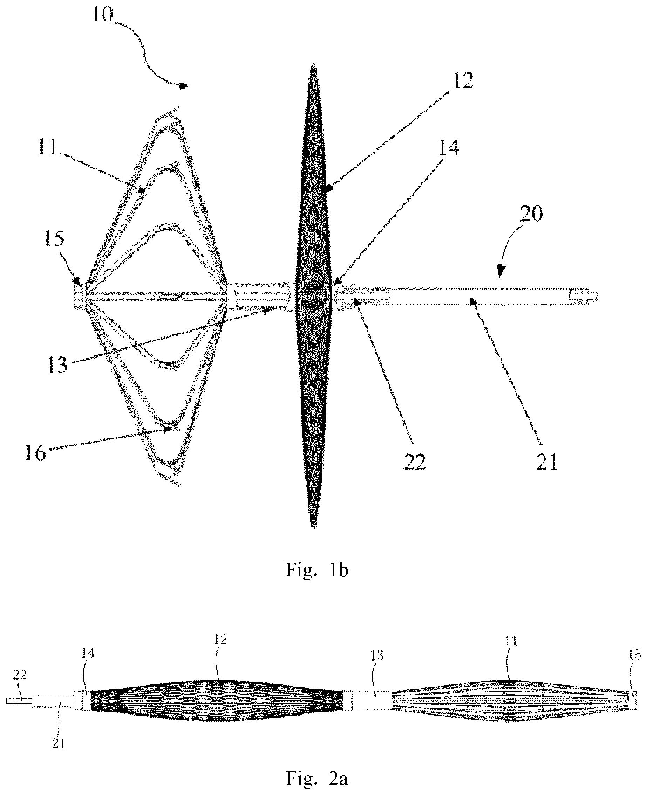

Fig. 1a is a schematic structural diagram of an occluder according to a preferred embodiment of the present invention; Fig. 1b is the front view of the medical system according to a preferred embodiment of the present invention. As shown in Figs. 1a and 1b, the embodiment provides a medical system, including an occluder 10 and a delivery device 20. The occluder 10 is configured to occlude the left atrial appendage. The delivery device 20 is configured to control the delivery, release and recovery of the occluder 10.

The occluder 10 includes a main body, which is entirely made of medically degradable materials. The main body includes a fixing disc 11 and an occluding disc 12. In another embodiment, only the fixing disc 11 is included, and no occluding disc 12 is included, and the main body of the occluder is only composed of the degradable fixing disc 11. The processing of the fixing disc 11 and the occluding disc 12 are not limited here, and each can be obtained by cutting or braiding. Generally, the fixing disc 11 is formed by cutting a degradable metal tube. In this case, the fixing disc 11 is a degradable metal stent to ensure the strength and good shaping ability of the fixing disc 11, but it is not limited to this. If degradable polymer materials can also achieve the same or similar effects, the fixing disc 11 can also be obtained by braiding or cutting. The occluding disc 12 is generally formed by braiding degradable polymer materials, and the occluding disc 12 is softer than the fixing disc 11, so that the compliance of the occluder is better. For example, the fixing disc 11 is optionally made of magnesium alloy, and the occluding disc 12 is optionally made of polylactic acid.

The occluder 10 also includes a hollow connection tube 13, a proximal fixing member 14, a distal fixing member 15 and an anchoring portion 16. The proximal end of the fixing disc 11 is connected to the distal end of the occluding disc 12 through a hollow connection tube 13. The hollow connection tube 13 has an inner cavity axially extending therethrough, and is preferably formed integrally with the fixing disc 11, such as integrally cut and formed, so as to simplify the structure and process. The proximal fixing member 14 is arranged at the proximal end of the occluding disc 12, and is preferably formed integrally with the occluding disc 12. If the main body of the occluder does not include an occluding disc 12, the proximal fixing member 14 is arranged at the proximal end of the fixing disc 11. The distal fixing member 15 is arranged at the distal end of the fixing disc 11, and is preferably formed integrally with the fixing disc 11. Optionally, the hollow connection tube 13 is made of magnesium alloy, the proximal fixing member 14 is made of polylactic acid, and the distal fixing member 15 is made of magnesium alloy. The anchoring portion 16 is connected to the fixing disc 11, preferably the anchoring portion 16 and the fixing disc 11 are integrally cut and formed.

The delivery device 20 includes an external push tube 21 and an internal push member 22. The internal push member 22 can be a rod capable of bearing axial compression, or a flexible member capable of bearing axial tension (such as wire, rope or thread, etc., preferably a guide wire). Relatively speaking, the size of the flexible body is smaller, which is beneficial to reduce the size of the delivery device 20. The flexible body can be detachably connected with the distal fixing member 15 in the manner of perforation, and there may be one or more perforations, and the axis of the perforation can be parallel or perpendicular to the axis of the fixing disc 11. The external push tube 21 is configured to detachably connect with the proximal fixing member 14 of the occluder 10 to achieve the purpose of pushing, adjusting and expanding the occluder 10. The internal push member 22 is configured to detachably connect with the distal fixing member 15 after passing through the external push tube 21, the proximal fixing member 14, the hollow connection tube 13, and the fixing disc 11 successively, so as to cooperate with the external push tube 21 to provide an axial pressure to the occluder 10, to cause the occluder 10 to expand radially. The connection mode between the external push tube 21 and the proximal fixing member 14 can be adjusted according to actual needs, such as selecting one or a combination of mechanical connections such as threaded connection, snap-in connection, clamp connection, perforation, or other non-mechanical connection modes. Similarly, the connection mode between the internal push member 22 and the distal fixing member 15 can be adjusted according to actual needs, and the detachable connection can be selected from one or a combination of mechanical connections such as threaded connection, snap-in connection, clamp connection, perforation, or other non-mechanical connection methods. In addition, the connection modes of the internal push member 22 and the external push tube 21 can be the same or different. For example, if a threaded connection is applied to the external push tube 21, a threaded connection or other connection mode can be applied to the internal push member 22, which is not limited here. In this embodiment, the external push tube 21 is connected the proximal fixing member 14 by threaded connection, and the internal push member 22 is connected to the distal fixing member 15 by threaded connection. For example, both the proximal fixing member 14 and the distal fixing member 15 have internal threads, and both the external push tube 21 and the internal push member 22 have external threads matching the internal threads.

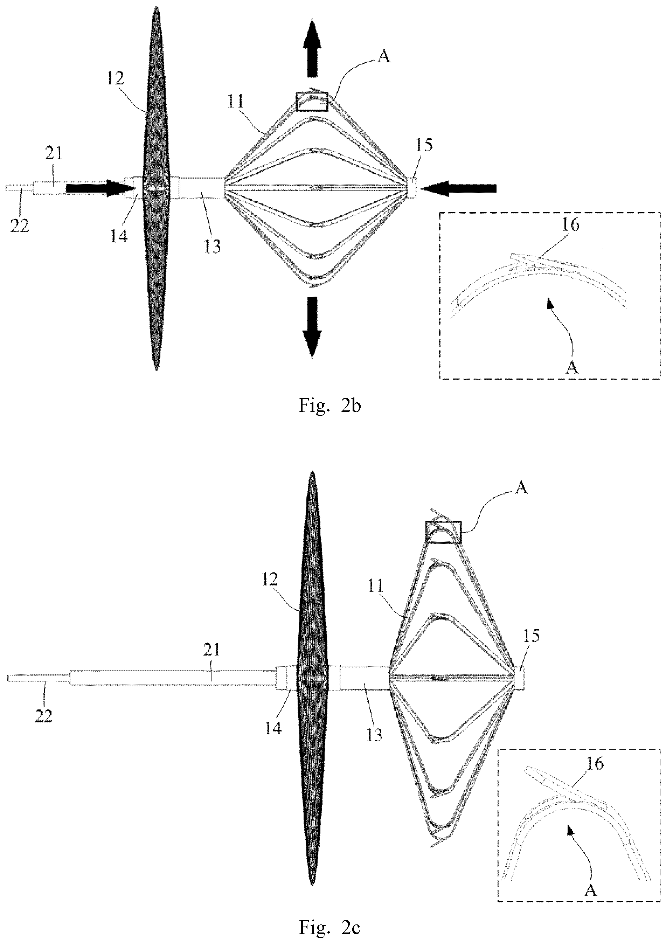

The occluder 10 has an initial state. In this state, the occluder 10 has not expanded. As shown in Fig. 2a, the occluder has a large axial dimension, and a small radial dimension. In other words, the occluder 10 has a collapsed configuration. The occluder 10 also has an expanded state, in which the occluder 10 is axially compressed and radially expanded, as shown in Figs. 2b and 2c. In practical application, the occluder 10 is pre-loaded in the sheath in the initial state and connected with the internal push member 22 and the external push tube 21; when the occluder 10 is delivered to the desired position and pushed out from the sheath, one of the external push tube 21 and the internal push member 22 is kept still, and the other moves in a predetermined direction, so that the occluder 10 expands from a collapsed configuration to an expanded configuration after being subjected to axial pressure, and during the expansion process of the occluder 10, the initially retracted anchoring portion 16 also gradually expands outwards, and finally penetrates the inner wall of the left atrial appendage; generally, when the occluder 10 is radially expanded to a predetermined size, and it is ensured that a stable connection is established between the occluder 10 and the left atrial appendage, the internal push member 22 and the external push tube 21 can be withdrawn successively to complete the occlusion of the left atrial appendage.

It should be understood that the traditional non-degradable anchoring portion has a shape memory function and can expand outwards autonomously, while the anchoring portion 16 of this embodiment is degradable and cannot expand outwards autonomously. Therefore, the expansion of the fixing disc 11 is required to help the anchoring portion 16 to expand outward, so that the anchoring portion 16 penetrates into the inner wall of the left atrial appendage. Furthermore, stabs (serving as the anchor portions 16) are formed by cutting the support rods 111 of the fixing disc 11, the support rods forming the stabs has a decreased width, so it is easier to be bent than other parts. When the occluder 10 switches from the collapsed configuration to the expanded configuration, it will naturally expand in the area where the anchoring portion 16 is located.

In more detail, as shown in Fig. 2a, after the occluder 10 is pushed out of the sheath, the occluder 10 is in the initial state if the internal push member 22 and the external push tube 21 are not operated. At this time, the fixing disc 11 and the occluding disc 12 do not expand, and the fixing disc 11 is positioned at the distal end of the occluding disc 12. As shown in Fig. 2b, the external push tube 21 and the internal push member 22 are operated so that for sample the external push tube 21 is kept still while the internal push member 22 moves proximally, or the internal push member 22 is kept still while the external push tube 21 moves distally, or the external push tube 21 and the internal push member 22 move toward each other simultaneously, any of which can make the occluder 10 to be compressed in the axial direction (indicated by the horizontal arrows in Fig. 2b), so that the axial length becomes shorter, and to be expand in the radial direction (indicated by the vertical arrows in Fig. 2b), so that the radial dimension becomes larger. At this time, with the expansion of the fixing disc 11, the anchoring portion 16 on the fixing disc 11 also expands outward; when the anchoring portion 16 expands outward, a positive angle is formed between the extension direction of the anchoring portion 16 and the positive direction of the axis of the occluder 10. The positive direction of the axis of the occluder is from the distal end to the proximal end of the occluder 10, and the extension direction (i.e., the length direction) of the anchoring portion 16 is from the tail end to the leading end of the anchoring portion 16. As shown in Fig. 2c, as the occluder 10 is further compressed, its radial dimension increases continuously, and the angle formed by the anchoring portion 16 also increases continuously, finally making the anchoring portion 16 face toward and penetrate the inner wall of the left atrial appendage. It should be understood that, in the dotted line frame at the lower right of Fig. 2b and Fig. 2c, an enlarged view of the schematic part A indicated by the solid line rectangle frame on the fixing disc 11 is also shown separately, so as to describe the state of the anchoring portion 16 on the fixing disc 11 as the fixing disc 11 expands.

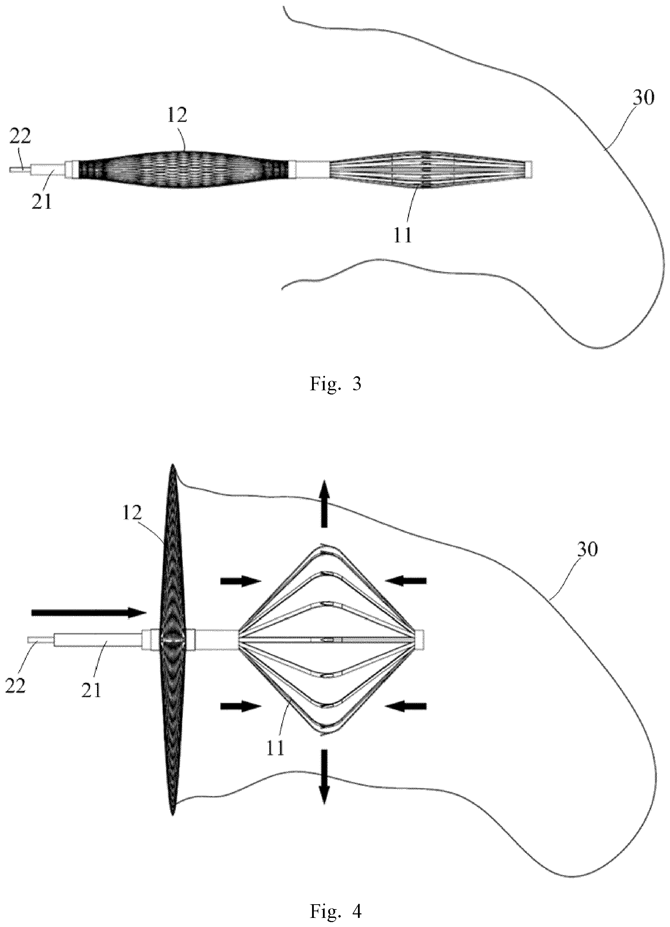

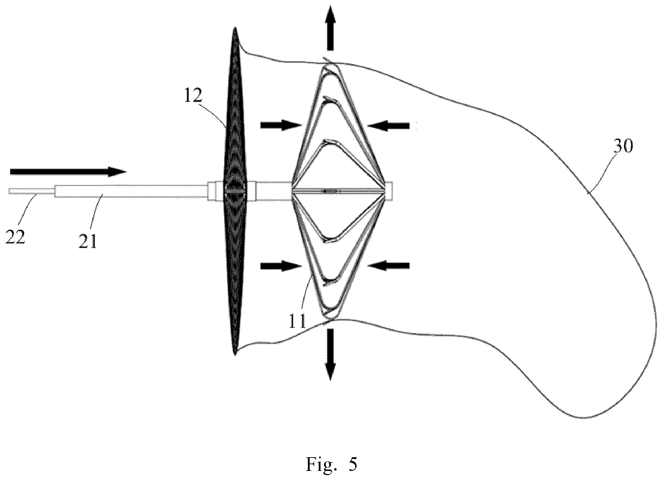

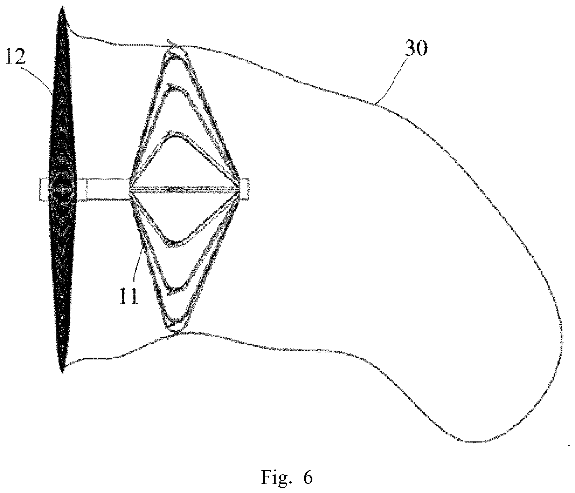

Further referring to Figs. 3 to 6, a preferred operation process of the occluder 10 of this embodiment will be further described. First, as shown in Fig. 3, when the occluder 10 is delivered to the desired position (so that for example the fixing disc 11 is located inside the left atrial appendage 30, and the occluding disc 12 is located outside the left atrial appendage 30 and in positional correspondence with the ostium of the left atrial appendage) and pushed out from the sheath. Before expansion, the occluder 10 can be moved or rotated by using the external push tube 21 or the internal push member 22 to adjust the position and angle of the occluder 10. After that, as shown in Figs. 4 and 5, after the completion of the adjustment on the occluder 10 in position and angle, the operator keeps the internal push member 22 still, and the external push tube 21 move distally, so that the occluder 10 expands from the collapsed configuration to the expanded configuration under pressure. During the expansion process, the anchoring portion 16 on the fixing disc 11 expands away from the axis of the fixing disc 11, and as the occluder 10 expands, the anchoring portion 16 gradually penetrates into the inner wall of the left atrial appendage, thereby establishing a stable and reliable connection. As further shown in Fig. 6, after the occluder 10 is successfully released, expanded and anchored, the operator withdraws the internal push member 22 and the external push tube 21 from the body in succession to complete the occlusion operation.

It should also be known that, for the double-disc occluder 10, the expansion sequence can be adjusted in practice. For example, the occluding disc 12 may be expanded first and then the fixing disc 11 may be expanded, or the fixing disc 11 may be expanded first and then the occluding disc 12 may be expanded, or the fixing disc 11 and the occluding disc 12 may be expanded at the same time, which may be controlled by factors such as material and size. For example, if the fixing disc 11 is softer than the occluding disc 12, after the occluder 10 is pushed out of the sheath, the fixing disc 11 first expands under axial compression, and the occluding disc 12 begins to expand only after the expansion of the fixing disc 11 is completed. If the occluding disc 12 is softer than the fixing disc 11, after the occluder 10 is pushed out of the sheath, the occluding disc 12 expands first under axial compression, and then the fixing disc 11 begins to expand only after the expansion of the occluding disc 12 is completed. If the the occluding disc 12 is as soft as the fixing disc 11, both the fixing disc 11 and the occluding disc 12 can expand simultaneously, that is, after the occluder 10 is pushed out of the sheath, the occluding disc 12 and the fixing disc 11 start to expand simultaneously under axial compression. Here, by adjusting the expansion sequence, it is convenient for doctors to operate. Generally, the occluding disc 12 is made by braiding degradable polymer materials, such as polylactic acid, and the fixing disc 11 is made by cutting degradable metal tubes, such as magnesium alloy. In doing so, the occluding disc 12 has the characteristics of softness and good compliance, which help the occluding disc 12 to better fit the ostium of the left atrial appendage, and thus improves the effectiveness of occlusion. Moreover, the soft occluding disc 12 is beneficial to reduce or even eliminate damage to the ostium of the left atrial appendage and nearby tissues, thereby improving the safety of occlusion. The use of the metal fixing disc 11 with increased strength is beneficial for occlusion and prevents it from falling out of the left atrial appendage.

Compared with the prior art, the occluder 10 of the present invention is subject to axial compression at the proximal and distal ends so that the main body of the occluder 10 expands radially to drive the anchoring portion 16 to stretch out and gradually penetrate into the inner wall of the left atrial appendage, so as to establish a stable connection to complete the occlusion. This structure overcomes the problem that the degradable occluder cannot self-expand to a predetermined shape after being pushed out of the sheath due to its material having no shape memory ability. Moreover, the protruding anchoring portion 16 can firmly connect with the left atrial appendage 30 during the compression, which solves the problem that the existing degradable left atrial appendage occluder cannot establish a reliable connection with the left atrial appendage.

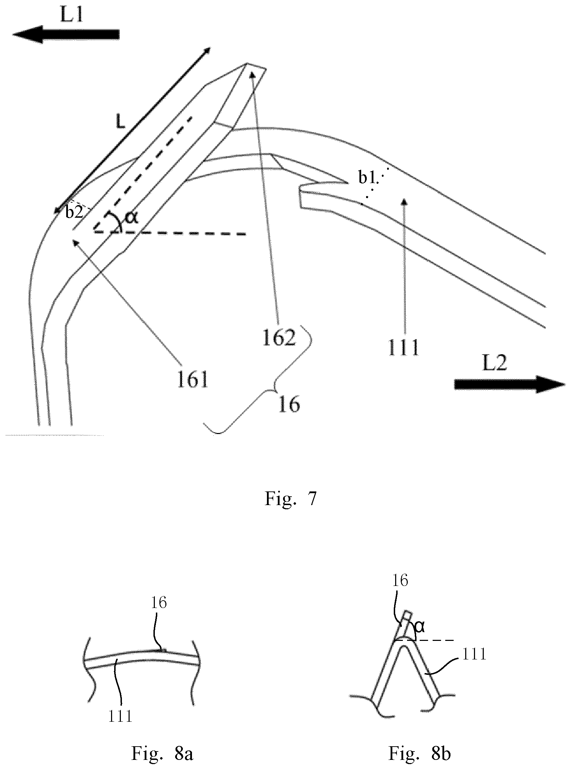

Further, as shown in Fig. 7, the anchoring portion 16 is preferably integrally cut and formed with the fixing plate 11. The fixing plate 11 includes a plurality of support rods 111 arranged circumferentially. At least some of the support rods 111 are cut at the middle part thereof to form an anchoring portion 16. In this embodiment, each of the support rods 111 is cut at the middle part thereof to form an anchoring portion 16. The anchoring portion 16 has a tail end 161 and a leading end 162 which are opposite with each other. The tail end 161 is connected to the fixing disc 11. The leading end 162 is a free end, and is preferably shaped as a pointed portion which is easier to penetrate into the inner wall of the left atrial appendage. Here, the support rods 111 and the anchoring portions 16 are simultaneously formed when the metal tube is cut, so the process is simpler and the manufacture is more convenient. During the practical operation, the support rods 111 can be obtained first (for example by cutting or braiding), and then the support rods 111 are cut to form the edge contours of the anchoring portions 16, and then the anchoring portions 16 are bent outward from the support rods 111.

Further considering that the strength of degradable materials is not as good as that of non-degradable materials such as nickel titanium, in some embodiments, it is preferable to cut from one side of the middle part of the support rod 111 to form the anchoring portion 16, so as to ensure the remainder of the support rod 111 to be as much as possible. Therefore, the strength of the fixing disc 11 is ensured. In some other embodiments, the width of the middle part of the support rod 111 can be designed to be greater than the width of the rest parts of the support rod 111. In this case, after the anchoring portion 16 is cut, the strength of the support rod 111 will not be reduced. In other embodiments, the width of the middle part of the support rod 111 is increased, and the support rod 111 can be cut from one side thereof. In addition, in other embodiments, the width of the middle part of the support rod 111 is increased, and the support rod 111 can be cut from one side of the support rod 111 or inside the support rod 111. In addition, it should be understood that the middle part of the support rod 111 should not be interpreted as an absolute middle position in a narrow sense, that is, the present invention does not limit the position of the middle part of the fixing disc 11 relative to the central axis, for example, the middle part may be at the central axis, or off the central axis. Referring to Figs. 1a and 1b, the anchoring portions 16 is mainly arranged at the middle part of the fixing disc 11 because the middle part of the fixing disc 11 bends outward under axial compression. The anchoring portion 16 is arranged at the part of the fixing disc 11 with the maximum outer diameter after expansion, so that the anchoring portion 16 can effectively stretch out and penetrate into the inner wall of the left atrial appendage.

Degradable metals, such as magnesium alloy, are not as strong as nickel-titanium alloy materials, and stress concentration is likely to occur at the bent portion. The above two reasons may cause the support rod 111 to break. In order to avoid this, firstly, the bending angle (curvature) should not be too large during the expansion process, and secondly, the stab can be formed by cutting from one side of the support rod, and the original width of the stab part of support rod before the stab is formed is slightly larger than the width of the rest parts of the support rod, and the width of the remainder of the support rod after the cutting is slightly smaller than the width of the rest parts of the support rod. Preferably, the width of the support rod 111 before the cutting is (1.1~1.3) b1 (as shown in Fig. 7, b1 refers to the width of the rest parts of the support rod other than the stab part), and/or the width b2 of the remainder of the support rod 111 after the cutting is (0.85~0.95) b1. It should be understood that the width of the support rod before the cutting refers to the width of the part of the support rod that needs to be cut to form the stab; in other words, before the stab is formed, the ratio of the width of the middle part of the support rod to the width of other parts is preferably 1.1:1-1.3:1. In addition, the ratio of the width of the remainder of the support rod after the cutting, which is the width of the middle part of the support rod without the stab, to the width of other parts is 0.85:1-0.95:1. The inventor found through many experiments and researches that designing the width of the support rod to the above-mentioned range can significantly improve the mechanical properties of the support rod and ensure the strength of the support rod.

As shown in Fig. 7, in the expanded state, an acute angle α is formed between the extension direction of the anchoring portion 16 and the positive direction of the axis of the fixing disc 11. The positive direction of the axis of the fixing disc 11 is from the distal end to the proximal end of the fixing disc 11. The extension direction of the anchoring portion 16 is the length direction which is from the tail end 161 to the leading end 162, wherein the arrow L1 directs to the distal end, and the arrow L2 directs to the distal end. It should be understood that, when the fixing disc 11 is expanded under pressure, the central part of the fixing disc 11 along the axial direction will be bent, and the curvature will gradually increase as the expansion progresses. In the expansion process, the tail end 161 (i.e., base) of the anchoring portion 16 is connected to the fixing disc 11, and the leading end 162 of the anchoring portion 16 is separated from the fixing disc 11, so the anchoring portion 16 does not bend accordingly. Therefore, during the expansion process of the fixing disc 11, the anchoring portion 16 initially retracted in the fixing disc 11 gradually stretches out, and penetrates into the inner wall of the left atrial appendage as the fixing disc 11 expands, establishing a stable connection with the inner wall of the left atrial appendage. Furthermore, if the length L and angle α of the anchoring portion 16 are too large, there may be risks such as pericardial effusion, tamponade, and device falling off. Therefore, it is necessary to control the shape and angle of the anchoring portion 16 after expansion. For this reason, the anchoring portion 16 is pre-bent before the expansion of the fixing disc 11.

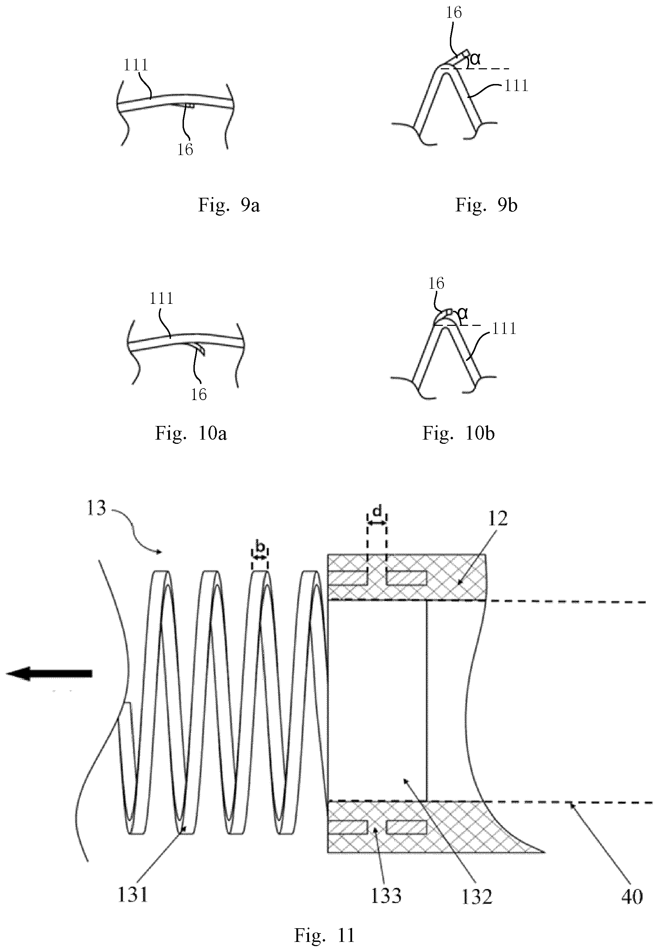

If the anchoring portion 16 is not pre-bent, the anchoring portion 16 does not stretch out before the expansion of the fixing disc 11 as shown in Fig. 8a. In this case, the angle α formed between the extension direction of the anchoring portion 16 and the positive direction of the axis of the fixing disc 11 may be 0° or slightly larger or smaller than 0°. In this way, when the anchoring portion 16 that has not been pre-bent stretches out after the expansion of the fixing disc 11 as shown in Fig. 8b, the angle α is large, causing the anchoring portion easy to detach from the inner wall of the left atrial appendage. In contrast, if the anchoring portion 16 is pre-bent to control the angle of the anchoring portion 16, the anchoring portion 16 is bent inward at a certain angle (negative angle) before the expansion of the fixing disc 11 as shown Fig. 9a. In this case, a negative angle is formed between the extension direction of the anchoring portion 16 and the positive direction of the axis of the fixing disc 11. Preferably, the anchoring portion 16 is pre-bent inward at an angle in the range of -50°∼-30°, more preferably, at an angle of -40°. As a result, although the anchoring portion 16 also stretches out after the expansion of the fixing disc 11 as shown in Fig. 9b, the angle α (positive angle) in this case is obviously smaller than that in the case where the anchoring portion 16 is not pre-bent. In addition, the anchoring portion 16 can also be pre-bent to control the shape and angle thereof. In this case, the anchoring portion 16 is bent inward at a certain angle (negative angle) and is preprocessed into an arc-shaped structure (as a hook) along the length direction before the expansion of the fixing disc 11 as shown in Fig. 10a. As shown in Fig. 10b, the angle α formed by the anchoring portion 16 which stretches out after the expansion of the fixing disc 11 is smaller than the angle α formed by the anchoring portion 16 which is not pre-bent, and the anchoring portion 16 is arc-shaped, which reduces effective length of the anchoring portion 16, wherein the effective length is the length of a straight line connecting the leading end 162 and the tail end 161 of the anchoring portion 16. Further, after the expansion of the fixing disc 11, the length L of the anchoring portion 16 is preferably 1.0 mm~2.0mm, the angle α of the anchoring portion 16 is preferably 20°~40°, more preferably 30°.

The shape and quantity of the anchoring portions 16 are not limited, and the anchoring portions 16 may be each shaped as a straight line, arc, curved line or folded line, and may be arranged in a single, double or more rows. The anchoring portion 16 has a shape including but not limited to a sheet shape.

As further shown in Fig. 11, the hollow connection tube 13 has preferably an elastic structure, such as an elastic cut tube or a coil spring. When the hollow connection tube 13 is configured to be elastic, the adaptability of the occluder 10 to different forms of left atrial appendages can be increased, especially the occlusion performance for occluding the left atrial appendage whose body and ostium are not coaxial is increased. In other words, the fixing disc 11 and the occluding disc 12 can be uncoaxial with each other based on elastic capacity of the hollow connection tube 13. In this embodiment, the hollow connection tube 13 is implemented as a spring, and is integrally cut and formed with the fixing disc 11. The spring has preferably implemented as a coil of 3 to 5 turns. If the spring is too long, the length of the entire occluder will be increased, and the axial distance between the fixing disc 11 and the occluding disc 12 will be increased, thereby increasing the risk of puncture of the left atrial appendage caused from the placement of the fixing disc 11 in the left atrial appendage at a deep position. If the spring is too short, the flexibility is not good enough. In addition, the axial width b of each turn of spring segment 131 can be selected as 0.3 mm-0.6mm.

In addition, the hollow connection tube 13 is connected to the occluding disc 12 preferably by hot melting, which provides a good connection effect and does not need to introduce external substances, and therefore can avoid safety problems such as complications caused by long-term implantation. Further, the proximal end of the hollow connection tube 13 is defined with a connecting portion 132 having a length of 1mm~2mm, and the distal end of the occluding disc 12 is connected with the connecting portion 132 by hot melting. In more detail, when the occluding disc 12 is connected to the hollow connection tube 13, the liner rod 40 with a diameter slightly smaller than the inner diameter of the hollow connection tube 13 is inserted into the connecting portion 132 of the hollow connection tube 13, and the distal end of the occluding disc 12 is sleeved over the connecting portion 132. Then, the distal end of the occluding disc 12 is heated at a temperature of 150°C ~ 210°C for a period of time of 5s ~ 15s, so as to ensure that the distal end is fully melted without affecting the remaining parts. During the hot melting process, the molten material (such as molten polylactic acid) of the occluding disc enters the hollow connection tube 13 through the hole 133 on the connecting portion 132 and fills the gap between the liner rod 40 and the connecting portion 132. After solidification, the occluding disc 12 is firmly connected with the fixing disc 11, and then the liner rod 40 is removed. The hole 133 on the connecting portion 132 extends through the hollow connection tube 13, and has a shape including but not limited to a circle. Generally, the size of the hole 133 is selected according to the inner diameter of the hollow connection tube 13. Optionally, the diameter d of the hole 133 is 0.5 mm~1.0 mm. The number and distribution of the holes 133 (including slots) are not limited.

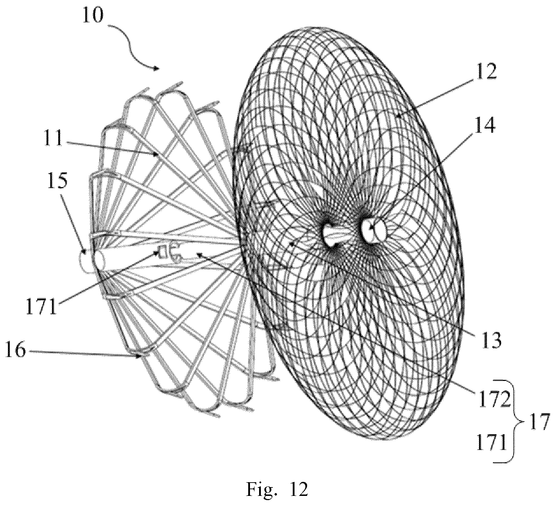

As shown in Fig. 12, the medical system preferably further includes a limiting device 17 configured to control the shape of the occluder during and after the expansion thereof, so as to control the size of the expanded occluder. When the occluder 10 expands to a predetermined size, the limiting device 17 locks the fixing disc 11 and the occluding disc 12, so that the occluder remains in the current configuration. Therefore, the size and shape of the expanded occluder are controlled. Optionally, the limiting device 17 includes a limiting slot 171 and a limiting fastener 172. When the occluder 10 is axially compressed to expand, the limiting slot 171 and the limiting fastener 172 are gradually approached. When the occluder 10 expands to a predetermined size, the limiting fastener 172 snaps into the limiting slot 171 to establish a fixed connection. The present invention does not limit the specific positions of the limiting slot 171 and the limiting fastener 172, which can be implemented in various ways. For example, in an embodiment, the limiting slot 171 is provided on the distal fixing member 15, and the distal fixing member 15 can be extended into the fixing disc 11 to form an extension. The limiting slot 171 is defined in the extension, or the extension can be connected with the distal fixing member 15. In addition, the limiting fastener 172 is arranged at the proximal end of the fixing disc 11, or the limiting fastener 172 is arranged on the hollow connection tube 13. Similarly, the distal end of the hollow connection tube 13 is extended into the fixing disc 11 to form an extension where the limiting fastener 172 is arranged. In addition, the positions of the limiting fastener 172 and the limiting slot 171 may be exchanged. Similarly, a limiting fastener 172 and a limiting slot 171 may be provided on the occluding disc 12. Moreover, those skilled in the art should know that the protection scope of the present invention is not limited by the manner in which the fastener cooperates with the slot in the embodiment to realize position limitation. It should be known that the limiting device 17 may be implemented in other forms, which are applicable to the present invention.

In the embodiments of the present application, the double-disc external-sealing occluder is taken as an example. Those skilled in the art should know that the protection scope of the present invention is not limited to the double-disc external-sealing occluder in the embodiment. It should be known that the cage-shaped occluder can also be implemented according to the description in the above-mentioned embodiments. Those skilled in the art should know that appropriate modifications can be made on the basis of the content disclosed in the above-mentioned embodiments to make the cage-shaped occluder achieve the same or similar effects. Compared with the double-disc occluder, the cage-shaped occluder dispenses with the occluding disc 12 and the hollow connection tube 13, and it is only required to provide the proximal fixing member 14 and the distal fixing member 15 respectively on the proximal end and the distal end of the fixing disc 11 (which is implemented as a cage-shaped stent formed by braiding or cutting). In this case, the proximal fixing member 14 is still connected with the external push tube 21, and the internal push member 22 is connected with the distal fixing member 15 by passing through the external push tube 21, the proximal fixing member 14 and the fixing disc 11 successively. The operation process of the cage-shaped occluder is basically the same as that of the double-disc occluder, which will not be described in detail here. In addition, a membrane may be provided on the occluding disc 12 and/or the fixing disc 11. After the delivery device 20 is withdrawn, the shape of the expanded occluder can be maintained by its own plastic deformation.

The above description is only for the description of preferred embodiments of the present invention, and is not intended to limit the scope of the present invention. Any changes and modifications made by those skilled in the art according to the above disclosure are all within the protection scope of the appended claims.

## Claims

1. A medical system, comprising a delivery device and a degradable occluder;
wherein the occluder comprises a main body, a proximal fixing member, a distal fixing member and an anchoring portion; wherein the main body has a proximal end connected to the proximal fixing member, and a distal end connected to the distal fixing member; the anchoring portion is defined on the main body;
wherein the delivery device comprises an external push tube and an internal push member; wherein the external push tube is configured to be detachably connected to the proximal fixing member; the internal push member is configured to pass through the external push tube, the proximal fixing member and the main body successively to detachably connect to the distal fixing member;
wherein the medical system is configured so that the delivery device applies an axial pressure to the occluder, to cause the main body to expand from a collapsed configuration to an expanded configuration to drive the anchoring portion to stretch out.

2. The medical system according claim 1, wherein the main body comprises a degradable metal stent and the anchoring portion integrally formed with the metal stent.

3. The medical system according to claim 2, wherein the metal stent comprises a plurality of support rods arranged circumferentially; wherein at least one of the support rods are cut at a middle part thereof to form the anchoring portion having a leading end and a tail end which are opposite to each other, wherein the tail end is connected to the metal stent, and the leading end is a free end;
wherein the anchoring portion is formed by cutting from one side of the middle part of the support rod, and/or the middle part of the support rod has a width larger than a width of rest parts of the support rod.

4. The medical system according to any one of claims 1-3, wherein the occluder has an initial state and an expanded state;
when the occluder is in the initial state, a negative angle is formed between an extension direction of the anchoring portion and a positive direction of an axis of the main body, and/or the a profile of the anchoring portion along a length direction thereof is arc-shaped;
when the occluder is in the expanded state, a positive angle is formed between the extension direction of the anchoring portion and the positive direction of the axis of the main body, and/or the profile of the anchoring portion along the length direction thereof is arc-shaped.

5. The medical system according to claim 4, wherein the negative angle ranges from -50° to -30°, and the positive angle ranges from 20° to 40°.

6. The medical system according to any one of claims 1-5, wherein the internal push member is a rod or a flexible member.

7. The medical system according to any one of claims 1-3, wherein the main body comprises a degradable metal stent and an occluding disc made by braiding degradable polymer material;
wherein the metal stent and the occluding disc are connected through a hollow connection tube; the anchoring portion is arranged on the metal stent; a distal end of the metal stent is connected to the distal fixing member, and a proximal end of the occluding disc is connected to the proximal fixing member.

8. The medical system according to claim 7, wherein the hollow connection tube is elastic.

9. The medical system according to claim 8, wherein the hollow connection tube is a spring integrally formed with the metal stent by cutting.

10. The medical system according to claim 7, wherein a proximal end of the hollow connection tube is defined with a connecting portion, and a hole extending through the connecting portion is defined in the connecting portion; the connecting portion is connected to a distal end of the occluding disc by hot melting.

11. The medical system according to claim 7, wherein the metal stent and the occluding disc expand simultaneously or sequentially after the occluder receives the axial pressure from the delivery device.

12. The medical system according to any one of claims 1-3, wherein the occluder is made of degradable metal material and/or degradable polymer material;
wherein the degradable metal material comprises at least one of magnesium-based metal, zinc-based metal and iron-based metal, and the degradable polymer material comprises polylactic acid, polydioxanone, polycaprolactone, polyglycolide, and polylactide-co-glycolide.

13. The medical system according to any one of claims 1-3, further comprising a limiting device configured to control a size of the expanded occluder.

14. The medical system according to claim 13, wherein the limiting device comprises a limiting fastener and a limiting slot; when the occluder expands to a predetermined size, the limiting slot engages with the limit fastener.

15. A medical device, made of degradable materials, the medical device comprising a degradable metal stent and an anchoring portion connected with the metal stent;
when the medical device is subjected to axial pressure, the metal stent expands from a collapsed configuration to an expanded configuration to drive the anchoring portion to stretch out.

16. The medical device according to claim 15, wherein the metal stent comprises a plurality of support rods arranged circumferentially; wherein at least one of the support rods are cut at a middle part thereof to form the anchoring portion having a leading end and a tail end which are opposite to each other, wherein the tail end is connected to the metal stent, and the leading end is a free end;
wherein the anchoring portion is formed by cutting from one side of the middle part of the support rod, and/or the middle part of the support rod has a width larger than a width of rest parts of the support rod.

17. The medical device according to claim 16, wherein the medical device has an initial state and an expanded state;
when the medical device is in the initial state, a negative angle is formed between an extension direction of the anchoring portion and a positive direction of an axis of the metal stent, and/or the a profile of the anchoring portion along a length direction thereof is arc-shaped;
when the medical device is in the expanded state, a positive angle is formed between the extension direction of the anchoring portion and the positive direction of the axis of the metal stent, and/or the profile of the anchoring portion along the length direction thereof is arc-shaped.

18. The medical device according to any one of claims 15-17, further comprising an occluding disc made by braiding degradable polymer material; wherein the metal stent and the occluding disc are connected through a hollow connection tube.

19. The medical device according to claim 16, wherein a ratio of the width of the middle part of the support rod to the width of the rest parts of the support rod is 1.1:1-1.3:1; and/or a ratio of a width of the middle part of the support rod without the anchoring portion to the width of the rest parts of the support rod is 0.85:1-0.95:1.
